# EUROPÄISCHE PATENTSCHRIFT

(11) **EP 2 284 531 B1**
(45) Veröffentlichungstag und Bekanntmachung des Hinweises auf die Patenterteilung: **25.09.2013**
(21) Anmeldenummer: 09166939.0
(22) Anmeldetag: 31.07.2009
(51) Int. Cl.: G01N 33/18, G01D 11/24, G01N 27/401

(54) **Wasseranalyse-Sensoranordnung**
Water analysis sensor assembly
Agencement de capteur pour l'analyse de l'eau

(43) Veröffentlichungstag der Anmeldung: 16.02.2011
(73) Patentinhaber: Hach Lange GmbH, 14163 Berlin (DE)
(72) Erfinder: Leyer, Axel, 41199 Mönchengladbach (DE); Heidemanns, Lothar, 41352 Korschenbroich (DE); Jonak, Andreas, 40667 Meerbusch (DE); Hahn, Dr. Markus, 47906 Kempen (DE); Kussmann, Michael, 40476 Düsseldorf (DE); Rudde, Heinz, 41836 Hückelhoven (DE); Rieger, Claudia, 40227 Düsseldorf (DE)
(74) Vertreter: Patentanwälte ter Smitten Eberlein Rütten Partnerschaftsgesellschaft

(56) Entgegenhaltungen:
- EP-A- 1 710 567
- DE-T5- 10 296 835
- US-A- 5 821 405
- US-A1- 2003 177 851

## Beschreibung

Die Erfindung bezieht sich auf eine Wasseranalyse-Tauchsonde mit mindestens zwei verschiedenen Sensoren zur Bestimmung verschiedener Wasser-Parameter.

Wasseranalyse-Tauchsonden dienen in der Regel zur Prozesssteuerung bei der Klärung oder Aufbereitung von Wasser oder zur kontinuierlichen Qualitätskontrolle von Wasser. Die Wasseranalyse-Tauchsonde weist hierzu verschiedene Sensoren auf, die der Bestimmung der gewünschten Parameter dienen bzw. Kompensation für einen Hauptparameter benötigt werden. Je nach Anwendung sind hierbei stets verschiedene Parameter bzw. Parameter-Kombinationen des Wassers zu bestimmen.

Für jede Parameter-Kombination ist daher eine individuelle Wasseranalyse-Tauchsonde zur Verfügung zu stellen.

Eine derartige Wasseranalyse-Tauchsonde ist beispielsweise aus EP 1 710 567 A1 bekannt. Hierbei weist die Tauchsonde eine Sensorkartusche auf, die drei Sensoren zur Bestimmung von drei verschiedenen Parametern aufweist. Für jede abweichende Kombination verschiedener Sensoren muss eine eigene Sensorkartusche gebaut werden.

Aus US 5,821,405 ist eine Wasseranalyse-Sonde bekannt, die eine Sondenbasis aufweist, die mehrere uniforme Sensormodul-Steckplätze aufweist, in die entsprechende Sensormodule eingesteckt und miteinander frei kombiniert werden können.

Aufgabe der Erfindung ist es demgegenüber, eine Wasseranalyse-Tauchsonde zu schaffen, die mit wenig Aufwand verschiedene Kombinationen verschiedener Sensoren zur Verfügung stellt.

Diese Aufgabe wird erfindungsgemäß gelöst mit den Merkmalen des Patentanspruches 1.

Die erfindungsgemäße Wasseranalyse-Tauchsonde weist eine Sensormodulaufnahme mit mindestens zwei Sensormodul-Steckplätzen auf, die physisch und elektrisch, das heißt mechanisch und bezüglich ihrer elektrischen Schnittstelle, gleichartig beziehungsweise identisch zueinander sind. Die Sensormodulaufnahme kann auch mehr als zwei, beispielsweise drei oder vier identische Sensormodul-Steckplätze aufweisen. Ferner sind mindestens zwei physisch baugleiche, das heißt von ihrer äußeren Form her weitgehend identische Sensormodule vorgesehen, die jeweils mit einem anderen Sensor versehen sind, wobei die Sensormodule in den Steckplätzen stecken beziehungsweise in diese einsteckbar sind. Auch die verschiedenen Sensormodule mit verschiedenen Sensoren weisen jeweils identische elektrische Schnittstellen für den elektrischen Anschluss an die Sensormodulaufnahme bzw. an die elektrische Schnittstelle des jeweiligen Sensormodul-Steckplatzes auf. Die Sensormodule mit verschiedenen Sensoren sind nur insoweit identisch miteinander, als dies für die Kompatibilität erforderlich ist. Das distale Ende eines Sensormoduls muss jedoch nicht stets gleichförmig ausfallen.

Unter physisch und elektrisch gleichartig ist vorliegend also eine Gleichartigkeit zu verstehen, die sicherstellt, dass auf jeden Sensormodul-Steckplatz jedes Sensormodul passt, nicht jedoch, dass die Sensormodule bezüglich ihres jeweiligen Sensors und der dahinter liegenden Chemie und Elektronik gleich ausgebildet sind.

In die Sensormodul-Steckplätze der Sensormodulaufnahme kann eine beliebige Kombination von Sensormodulen mit verschiedenen Sensoren eingesteckt werden. Auf diese Weise kann für jede Anwendung auf einfache Weise die gewünschte Kombination an Sensoren für eine Wasseranalyse-Tauchsonde zusammengestellt werden. Die gewünschte Kombination an Sensoren kann sowohl durch den Hersteller als auch durch den Kunden zusammengestellt beziehungsweise zusammengesteckt werden.

Die Sensormodulaufnahme weist eine Referenzelektrode und einen Elektrolyttank für die Referenzelektrode auf.

Das Sensormodul kann einen Redox-, einen Chlorid-, einen Nitrat, einen Kalium-, einen Enzym-, einen Ammonium- oder einen pH- Sensor aufweisen bzw. eine entsprechende oder eine andere ionenselektive Membran aufweisen.

Vorzugsweise weist die Sensormodulaufnahme einen Temperatursensor auf. Alternativ oder ergänzend kann die Sensormodulaufnahme ferner einen Stromschlüssel, beispielsweise in Form einer sog. Salzbrücke, und/oder eine Masseelektrode aufweisen.

An der Sensormodulaufnahme sind bevorzugt alle Sensoren und Mittel vorgesehen, die im Zusammenhang mit den meisten unter allen verschiedenen Sensoren ohnehin stets benötigt werden. Auf diese Weise kann die Anzahl der Steckplätze und der dafür erforderliche Aufwand, insbesondere der erforderliche Bauraum und die erforderlichen Mittel für Energie- und Informationsübertragung, auf das unbedingt erforderliche beschränkt werden.

Jedes Sensormodul weist einen elektronischen Sensorinformations-Speicher mit Informationen über den betreffenden Sensor auf. In dem Sensorinformations-Speicher können sowohl Informationen über die Art des Sensors als auch individuelle Informationen über den Sensor, beispielsweise Kalibrierdaten, gespeichert sein. Ferner weist jedes Sensormodul ein Übertragungsmittel zur Übertragung der Sensorinformationen aus dem Sensorinformations-Speicher zu der Sensormodulaufnahme auf. Das Übertragungsmittel kann von einem drahtlosen Sender und/ oder alternativ beziehungsweise ergänzend von elektrischen Kontakten gebildet werden, die mit einem entsprechenden Übertragungsmittel an dem Sensormodul-Steckplatz auf der Seite der Sensormodulaufnahme zusammenwirken. Das Sensormodulaufnahme-Übertragungsmittel kann beispielsweise ein entsprechender drahtloser Sender-Empfänger-Baustein sein

Ferner weist jedes Sensormodul, soweit erforderlich, eine individuelle Innenausstattung auf, die zum Betrieb des betreffenden Sensors und zur Auswertung des Sensorsignals erforderlich ist. Diese Innenausstattung kann gegebenenfalls aus einem Tank mit einem Elektrolyt oder einer anderen Flüssigkeit, einer Mikropumpe, einem Messverstärker, einer Auswerteelektronik etc. bestehen.

Vorzugsweise ist das Sensormodul als Einwegteil ausgebildet. Das Einwegteil-Sensormodul wird nach seiner Erschöpfung beziehungsweise bei Auftreten einer Fehlfunktion entfernt und weggeworfen, und durch ein neues entsprechendes Sensormodul ersetzt.

Im Folgenden wird unter Bezugnahme auf die Zeichnungen ein Ausführungsbeispiel der Erfindung näher erläutert.

Es zeigen:
- Figur 1: eine Wasseranalyse-Tauchsonde, bestehend aus einer Sensormodulaufnahme und vier einsteckbaren Sensormodulen, und
- Figur 2: die Wasseranalyse-Tauchsonde der Figur 1 einschließlich eingesteckter Sensormodule in schematischer Darstellung.

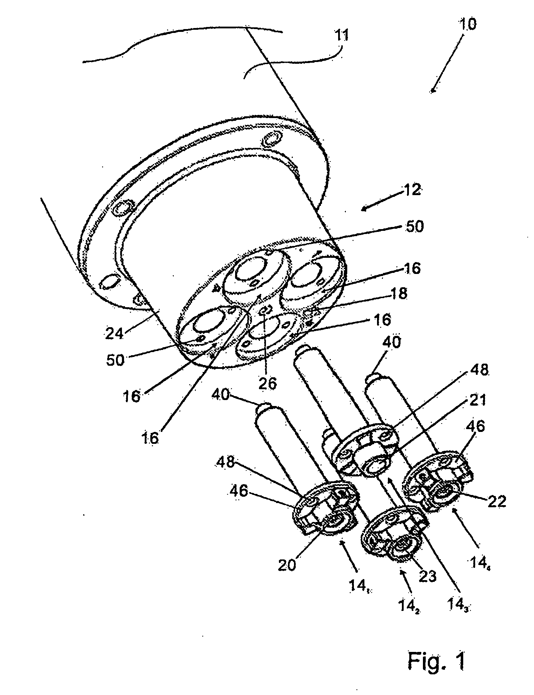

In Figur 1 ist in perspektivischer Darstellung eine Wasseranalyse-Tauchsonde 10 zur Bestimmung von vier verschiedenen Wasser-Parametern dargestellt. Die Tauchsonde 10 besteht im Wesentlichen aus einer Sondenbasis 11 und einer austauschbaren Sensormodulaufnahme 12, in die vier einzelne Sensormodule 14₁,14₂,14₃,14₄ einsteckbar sind. Die Sensormodulaufnahme 12 weist hierzu vier identische Steckplätze 16 auf, in die die äußerlich weitgehend identischen vier Sensormodule 14₁,14₂,14₃,14₄ eingeschoben werden können bzw. eingeschoben sind.

Die vier Sensormodule 14₁,14₂,14₃,14₄ sind bezüglich ihrer Sensoren 20,21,22,23 am distalen Sensormodul-Ende verschieden. Die Sensoren 20,21,22,23 können jeweils eine Redox-, eine Chlorid-, eine Nitrat-, eine Kalium-, eine Enzym-, eine Ammonium-, eine pH- oder eine andere, beispielsweise eine ionenselektive Elektrode sein. Die Elektroden der Sensoren 20,21,22,23 können beispielsweise als Metallelektrode, Festkörpermembranelektrode und/oder PVC-Membranelektrode ausgebildet sein.

Die Sensormodule 14₁,14₂,14₃,14₄ sind jeweils Einwegteile, werden also nur einmal eingesetzt, und nach Erschöpfung oder einem. Defekt entsorgt. Jedes Sensormodul 14₁,14₂,14₃,14₄ weist jeweils eine Verdrehsicherung auf, die mit einem entsprechenden Gegenstück an dem Steckplatz 16 verdrehsichernd zusammenwirkt. Ferner weist jedes Sensormodul 14₁,14₂,14₃,14₄ an seinem distalen Ende einen ringförmigen Befestigungsflansch 46 auf, der wiederum drei Befestigungsbohrungen 48 aufweist Die Befestigung eines Sensormoduls 14₁,14₂,14₃,14₄ wird durch entsprechende Gewindeschrauben vorgenommen, die in die Befestigungsbohrungen 48 eingesteckt und in entsprechende Gewindebohrungen 50 an der Stirnseite der Sensormodulaufnahme 12 eingeschraubt werden. Zwischen dem Befestigungsflansch 46 und der Sensorbasis 12 ist ein Dichtring vorgesehen, der für eine flüssigkeitsdichte Abdichtung des Ringraumes um das Sensormodul 14₁,14₂,14₃,14₄ herum in dem Steckplatz 16 sorgt.

An der Stirnseite der Sensormodulaufnahme 12 ist ein Stromschlüssel 18 vorgesehen, beispielsweise in Form einer so genannten Salzbrücke. Das Gehäuse 24 der Sensormodulaufnahme 12 und eine in einer Querebene liegende und in Längsrichtung im Bereich der Steckplätze 18 liegende Tankwand 31 bilden einen Elektrolyttank 30 für ein Elektrolyt, das mit dem Stromschlüssel 18 und einer von oben eingetauchten Referenzelektrode in Kontakt steht. Ferner weist die Sensormodulaufnahme 12 an ihrer Stirnseite einen Temperatursensor 26 auf, und kann ferner noch eine nicht dargestellte Masseelektrode aufweisen.

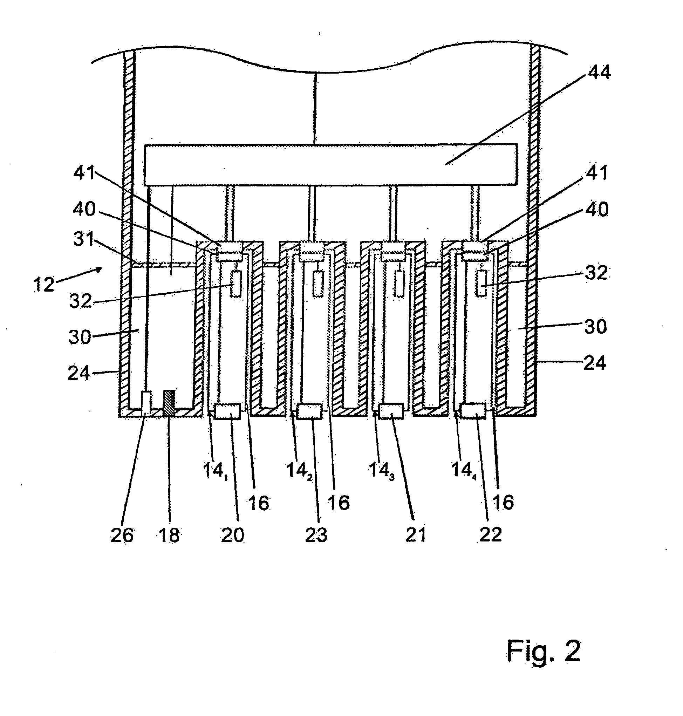

Wie in der schematischen Darstellung der Figur 2 dargestellt, weist jedes Sensormodul 14₁,14₂,14₃,14₄ einen elektronischen Sensorinformations-Speicher 32 auf, in dem Informationen über den betreffenden Sensor 20, 21,22, 23 gespeichert sind, beispielsweise die Sensorart und Kalibrierungs-Informationen.

Jedes Sensormodul 14₁,14₂,14₃,14₄ weist zur Übertragung der Sensor-Informationen, zu denen neben den Informationen aus dem Sensorinformations-Speicher 32 auch das Messsignal des betreffenden Sensors gehört, als Übertragungsmittel elektrische Kontakte 40 auf, die bei in den Steckplatz 16 eingestecktem Sensormodul 14₁,14₂,14₃,14₄ mit entsprechenden elektrischen Kontakten 41 des Steckplatzes 16 kontaktiert sind. Die elektrischen Kontakte 41 der Steckplätze 16, der Temperatursensor 26 und die Salzbrücke 18 über das Elektrolyt in dem Elektrolyttank 30 sind elektrisch mit einer zentralen Steuereinrichtung 44 verbunden, die die Steuerung und Kontrolle aller Bauteile und die Auswertung der Sensorsignale vornimmt.

Alternativ zu elektrischen Kontakten können die Energie und die Informationen auch drahtlos über entsprechende Spulen beziehungsweise Sender und Empfänger übertragen werden. Das Übertragungsmittel ist in dem Fall ein drahtloser Sender.

Die Konfiguration der Wasseranalyse-Tauchsonde 10 für eine bestimmte Anwendung beziehungsweise für eine bestimmte Kombination von zu bestimmenden Wasser-Parametern kann durch entsprechende Auswahl und Montage der Sensormodule 14₁,14₂,14₃,14₄ an der Sensormodulaufnahme 12 vorgenommen werden. Die Informationen über die Art des betreffenden Sensors 20,21,22,23 werden von der Steuereinrichtung 44 aus den betreffenden Sensorinformations-Speichern 32 ausgelesen, so dass Verwechslungen beziehungsweise manuelle Fehleingaben praktisch ausgeschlossen sind.

## Patentansprüche

1. Wasseranalyse-Tauchsonde (10) mit mindestens zwei Sensoren (20,21,22,23) zur Bestimmung verschiedener Wasser-Parameter, mit
einer Sensormodulaufnahme (12) mit mindestens zwei Sensormodul-Steckplätzen (16), die physisch und elektrisch gleichartig sind, und
mindestens zwei Sensormodulen (14₁, 14₂,14₃, 14₄) mit jeweils einem Sensor (20,21,22,23) wobei die Sensormodule (14₁, 14₂,14₃, 14₄) in den Steckplätzen (16) stecken, wobei die Sensonnodulaufnahme (12) austauschbar an einer Sondenbasis (11) befestigt ist,
wobei die Sensormodulaufnahme (12) eine Referenzelektrode und einen Elektrolyttank für die Referenzelektrode aufweist.

2. Wasseranalyse-Tauchsonde (10) nach Anspruch 1, wobei die Sensormodulaufnahme (12) einen Stromschlüssel (18) aufweist.

3. Wasseranalyse-Tauchsonde (10) nach Anspruch 2, wobei der Stromschlüssel (18) mit dem Elektrolyt des gefüllten Elektrolyttanks in Kontakt steht.

4. Wasseranalyse-Tauchsonde (10) nach einem der vorangegangenen Ansprüche, wobei die Sensormodulaufnahme (12) einen Temperatursensor (26) aufweist.

5. Wasseranalyse-Tauchsonde (10) nach einem der vorangegangenen Ansprüche, wobei die Sensormodulaufnahme (12) eine Masseelektrode aufweist.

6. Wasseranalyse-Tauchsonde (10) nach einem der Ansprüche 1 bis 5, wobei der Elektrolyttank (30) von einem Gehäuse (24) der Sensormodulaufnahme (12) gebildet wird.

7. Wasseranalyse-Tauchsonde (10) nach einem der vorangegangenen Ansprüche, wobei jedes Sensormodul (14₁,14₂,14₃,14₄) einen elektronischen Sensorinformations-Speicher (32) mit Informationen über den betreffenden Sensor (20,21,22,23) und ein Übertragungsmittel zur Übertragung der Sensorinformation zu der Sensormodulaufnahme (12) aufweist.

8. Wasseranalyse-Tauchsonde (10) nach Anspruch 7, wobei das Übertragungsmittel ein drahtloser Sender ist.

9. Wasseranalyse-Tauchsonde (10) nach Anspruch 7, wobei das Übertragungsmittel von elektrischen Kontakten (41,40) an dem Steckplatz (16) einerseits und an dem Sensormodul (14₁,14₂,14₃,14₄) andererseits gebildet wird.

10. Wasseranalyse-Tauchsonde (10) nach einem der vorangegangenen Ansprüche, wobei das Sensormodul (14₁, 14₂,14₃,14₄) einen Redox-, eine Chlorid-, eine Nitrat, eine Kalzium-, Enzym-, eine Ammonium-, einen pH- Sensor (20,21,22,23) oder eine ionenselektive Elektrode aufweisen kann.

11. Wasseranalyse-Tauchsonde (10) nach einem der vorangegangenen Ansprüche, wobei das Sensormodul (14₁, 14₂,14₃,14₄) ein Einwegteil ist.

## Claims

1. Submersible water analysis probe (10) having at least two sensors (20, 21, 22, 23) for the determination of various water parameters, comprising
a sensor module receptacle (12) having at least two sensor module slots (16) that are physically and electrically similar, and
at least two sensor modules (14₁, 14₂, 14₃, 14₄), each having a sensor (20, 21, 22, 23), the sensor modules (14₁, 14₂, 14₃, 14₄) sitting in the sensor slots (16), the sensor module receptacle (12) being mounted to a probe base (11) in a replaceable manner,
wherein the sensor module receptacle (12) comprises a reference electrode and an electrolyte tank for the reference electrode.

2. Submersible water analysis probe (10) of claim 1, wherein the sensor module receptacle (12) comprises a salt bridge (18).

3. Submersible water analysis probe (10) of claim 2, wherein the salt bridge (18) is in contact with the electrolyte of the filled electrolyte tank.

4. Submersible water analysis probe (10) of one of the preceding claims, wherein the sensor module receptacle (12) comprises a temperature sensor (26).

5. Submersible water analysis probe (10) of one of the preceding claims, wherein the sensor module receptacle (12) comprises a mass electrode.

6. Submersible water analysis probe (10) of one of claims 1 to 5, wherein the electrolyte tank (30) is formed by a housing (24) of the sensor module receptacle (12).

7. Submersible water analysis probe (10) of one of the preceding claims, wherein each sensor module (14₁, 14₂, 14₃, 14₄) includes an electronic sensor information memory (32) with information about the respective sensor (20, 21, 22, 23) and a transmission means for the transmission of the sensor information to the sensor module receptacle (12).

8. Submersible water analysis probe (10) of claim 7, wherein the transmission means is a wireless transmitter.

9. Submersible water analysis probe (10) of claim 7, wherein the transmission means is formed by electric contacts (41, 40) at the slot (16), on the one hand, and at the sensor module (14₁, 14₂, 14₃, 14₄), on the other hand.

10. Submersible water analysis probe (10) of one of the preceding claims, wherein the sensor module (14₁, 14₂, 14₃, 14₄) can include a redox-, a chloride-, a nitrate-, a potassium-, an enzyme-, an ammonium-, a pH-sensor (20, 21, 22, 23) or an ion-selective electrode.

11. Submersible water analysis probe (10) of one of the preceding claims, wherein the sensor module (14₁, 14₂, 14₃, 14₄) is a single-use element.

## Revendications

1. Sonde immersible (10) pour l'analyse d'eau avec au moins deux capteurs (20, 21, 22, 23) pour la détermination de diverses paramètres d'eau, comprenant
un support de module capteur (12) avec au moins deux logements de module capteur (16), qui sont similaires physiquement et électriquement, et
au moins deux modules capteur (14₁, 14₂, 14₃, 14₄), chacun avec un capteur (20, 21, 22, 23), lesdits modules capteur (14₁, 14₂, 14₃, 14₄) étant insérés dans les logements (16), ledit support de module capteur (12) étant monté de façon interchangeable sur une embase de sonde (11),
ledit support de module capteur (12) comprenant une électrode de référence et un réservoir à électrolyte pour ladite électrode de référence.

2. Sonde immersible (10) pour l'analyse d'eau selon la revendication 2, dans lequel ledit support de module capteur (12) comprend un pont électrolytique (18).

3. Sonde immersible (10) pour l'analyse d'eau selon la revendication 2, dans lequel ledit pont électrolytique (18) est en contact avec l'électrolyte dudit réservoir à électrolyte rempli.

4. Sonde immersible (10) pour l'analyse d'eau selon l'une quelconque des revendications précédentes, dans lequel ledit support de module capteur (12) comprend un capteur de température (26).

5. Sonde immersible (10) pour l'analyse d'eau selon l'une quelconque des revendications précédentes, dans lequel ledit support de module capteur (12) comprend une électrode de masse.

6. Sonde immersible (10) pour l'analyse d'eau selon l'une quelconque des revendications 1 à 5, dans lequel ledit réservoir à électrolyte (30) est formé par un boitier (24) dudit support de module capteur (12).

7. Sonde immersible (10) pour l'analyse d'eau selon l'une quelconque des revendications précédentes, dans lequel chaque module capteur (14₁, 14₂, 14₃, 14₄) comprend une mémoire (32) pour informations de capteurs stockant informations sur un capteur (20, 21, 22, 23) respectif, et un moyens de transmission pour transmettre les informations de capteur audit support de module capteur (12).

8. Sonde immersible (10) pour l'analyse d'eau selon la revendication 7, dans lequel le moyen de transmission est un émetteur sans fil.

9. Sonde immersible (10) pour l'analyse d'eau selon la revendication 7, dans lequel ledit moyen de transmission est constitué par des bornes électriques (41, 42) au logement (16), d'un part, et au module capteur (14₁, 14₂, 14₃, 14₄), d'autre part.

10. Sonde immersible (10) pour l'analyse d'eau selon l'une quelconque des revendications précédentes, dans lequel ledit module capteur (14₁, 14₂, 14₃, 14₄) peut comprendre un capteur de redox, de chlorure, de nitrate, de potassium, d'enzyme, d'ammonium, de pH (20, 21, 22, 23) ou une électrode sélective d'ions.

11. Sonde immersible (10) pour l'analyse d'eau selon l'une quelconque des revendications précédentes, dans lequel ledit module capteur (14₁, 14₂, 14₃, 14₄) est un élément jetable.
